# FASCICULE DE BREVET EUROPEEN

(11) **EP 0 593 348 B1**
(45) Date de publication et mention de la délivrance du brevet: **25.03.1998**
(21) Numéro de dépôt: 93402497.7
(22) Date de dépôt: 11.10.1993
(51) Int. Cl.: C07D 277/34, C07D 417/10, C07D 417/12, A61K 31/425

(54) **Dérivés de thiazolidin-2,4-dione, leur procédé de préparation et les compositions pharmaceutiques les contenant**
Thiazolidin-2,4-dion-Derivate, Verfahren zu ihrer Herstellung und pharmazeutische Zusammensetzungen die sie enthalten
Thiazolidin-2,4-dione derivatives, process for their preparation and pharmaceutical compositions containing them

(30) Priorité: 12.10.1992 FR 9212123
(43) Date de publication de la demande: 20.04.1994
(73) Titulaire: ADIR ET COMPAGNIE, 92415 Courbevoie Cédex (FR)
(72) Inventeur: Regnier, Gilbert, F-92290 Chatenay Malabry (FR); Charton, Yves, F-92330 Sceaux (FR); Duhault, Jacques, F-78290 Croissy sur Seine (FR); Espinal, Joseph, F-75008 Paris (FR)
(74) Mandataire: Reverbori, Marcelle

(56) Documents cités:
- EP-A- 0 008 203
- EP-A- 0 207 581
- EP-A- 0 332 331
- EP-A- 0 332 332
- EP-A- 0 389 699
- WO-A-92/07850
- CHEMICAL AND PHARMACEUTICAL BULLETIN. vol. 30, no. 10 , Octobre 1982 , TOKYO JP pages 3580 - 3600 TAKASHI SOHDA ET AL 'Studies on antidiabetic agents.II.Synthesis of 5-[4-( 1-methylcyclohexylmethoxy)-benzyl]thiazoli dine-2,4-dione(ADD-3878) and its derivatives'
- JOURNAL OF MEDICINAL CHEMISTRY vol. 35, no. 10 , 15 Mai 1992 , WASHINGTON US pages 1853 - 1864 BERNARD HULIN ET AL 'Novel thiazolidine-2,4-diones as potent euglycemic agents'

## Description

Elle concerne plus particulièrement :
- les composés de thiazolidine dione de formule I : 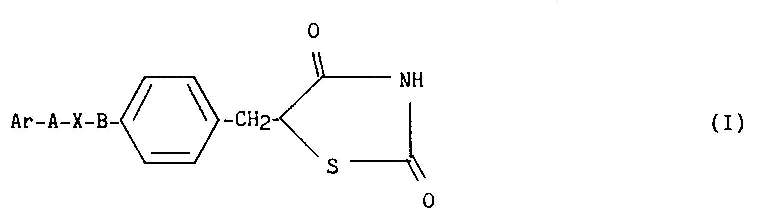 dans laquelle :
   Ar représente :
      un radical hétérobicyclique renfermant 1 ou 2 hétéroatomes choisis parmi les atomes d'azote, d'oxygène ou de soufre, tel que par exemple les radicaux indolyle ou chromanyle, chacun éventuellement substitué par un radical oxo- et/ou par un ou plusieurs atomes d'halogène, radicaux alkyle (ayant de 1 à 5 atomes de carbone) ou phényle lui-même éventuellement substitué par un ou plusieurs atomes d'halogène, radicaux trifluorométhyle, alkyle ou alkoxy ayant chacun de 1 à 5 atomes de carbone ;
   A représente : une liaison simple, une chaîne hydrocarbonée de 2 à 3 atomes de carbone renfermant une double liaison, ou une chaîne de formule -(CH₂)m- , 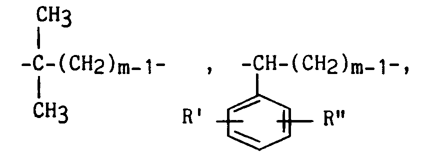 -O-(CH₂)ₘ-, ou -S-(CH₂)ₘ-, dans lesquelles :
      - m est un nombre entier de 1 à 3 et R' et R'', identiques ou différents, représentent chacun un atome d'hydrogène ou un radical alkyle ayant de 1 à 5 atomes de carbone en chaîne droite ou ramifiée ;
   X représente -CONR- ou -SO₂NR- dans lesquelles R représente un atome d'hydrogène ou un radical alkyle de 1 à 5 atomes de carbone en chaine droite ou ramifiée renfermant éventuellement une double liaison;
   B représente une chaine hydrocarbonée saturée de 1 à 6 atomes de carbone éventuellement ramifiée et/ou substituée par un radical hydroxy ou un radical oxo ;
ainsi que les énantiomères et dans le cas où B est ramifié, les diastéréoisomères correspondants.

L'état antérieur de la technique est illustré notamment par les documents suivants: Chem. Pharm. Bull. (1982), 30, 3580 ; J. Med. Chem. (1992), 35, 1853 ; EP-A-332331 ; EP-A-332332 ; EP-A-389699 ; EP-A-8203 ; EP-A-207581; WO-A-92/07850; WO-A-86/07056 et WO-A-85/04171 lesquels concernent des composés de thiazolidine-2,4-dione utilisables comme agents antidiabétiques.

Or les composés de la présente invention, outre le fait qu'ils soient nouveaux, se différencient des composés de thiazolidine-2,4-dione antérieurement connus par l'intensité de leurs propriétés pharmacologiques.

En effet, l'insulino-résistance et le défaut de sécrétion de l'insuline sont les responsables de l'intolérance au glucose observée chez les patients ayant un diabète non insulino-dépendant.

Les thérapeutiques actuellement disponibles permettent de corriger essentiellement le défaut de sécrétion d'insuline sans nécessairement améliorer la sensibilité à l'insuline des tissus périphériques (muscles, tissu adipeux).

Les composés de thiazolidine-2,4-dione sont capables de provoquer une baisse de la glycémie et d'améliorer la torérance au glucose dans les modèles de diabète non insulino-dépendant sans provoquer une augmentation de la sécrétion d'insuline.

Les composés de la présente invention ont l'avantage d'être particulièrement puissants (plus spécialement par rapport à la ciglitazone, composé de référence appartenant à cette classe chimique) tout en étant dépourvus d'effets hématologiques nocifs, comme le prouve l'étude pharmacologique ci-après décrite.

Ainsi les composés de la présente invention sont utilisables dans le traitement des états d'insulino-résistance et/ou des états diabétiques non insulinopéniques, permettant d'obtenir un meilleur contrôle de la glycémie alors que le taux d'insuline circulante diminue. La prévention de cette hyperinsulinémie relative, associée à une diminution des triglycérides circulant sous l'effet de ces produits, peut concourir à une réduction des risques de macroangiopathie.

Ces mêmes composés trouvent de plus une utilisation dans le traitement de l'hypertension chez les sujets âgés présentant une insulino-résistance associée ou non à d'autres anomalies métaboliques.

La présente invention a également pour objet les compositions pharmaceutiques contenant comme principe actif un composé de formule I ou un de ses sels physiologiquement tolérable, mélangé ou associé à un ou plusieurs excipients pharmaceutiques appropriés.

Les compositions pharmaceutiques ainsi obtenues se présentent généralement sous forme dosée renfermant de 100 à 200 mg de principe actif. Elles peuvent, par exemple, revêtir la forme de comprimés, dragées, gélules, suppositoires, solutions injectables ou buvables et être administrées par la voie orale, rectale ou parentérale.

La posologie peut varier notablement selon l'âge et le poids du patient, la voie d'administration, la nature de la maladie et les traitements associés et s'échelonne de 100 à 200 mg de principe actif, 2 à 3 fois par jour.

La présente invention a aussi pour objet le procédé de préparation des composés de formule I
caractérisé en ce que :
- soit,
   A) l'on fait réagir un composé de formule II : 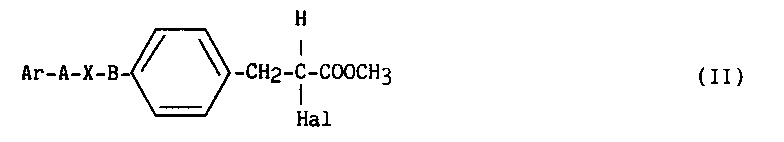 dans laquelle :
      - Ar, A, X et B ont les significations précédemment définies et
      - Hal représente un atome de chlore ou de brome,
      avec la thiourée 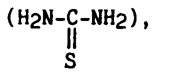 et l'on hydrolyse l'imine ainsi obtenue de formule III : 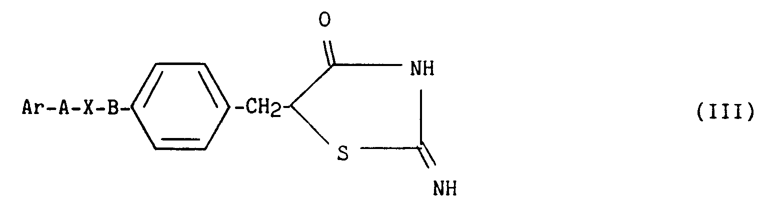 dans laquelle Ar, A, X et B ont les significations précédemment définies ;
- soit,
   B) l'on fait réagir un composé de formule IV :

      Ar-A-X' (IV)

      dans laquelle :
      - Ar et A ont les significations précédemment définies, et
      - X' représente COCl, SO₂Cl, un ester activé ou CO-O-CO-A-Ar,
      avec un composé de formule V : 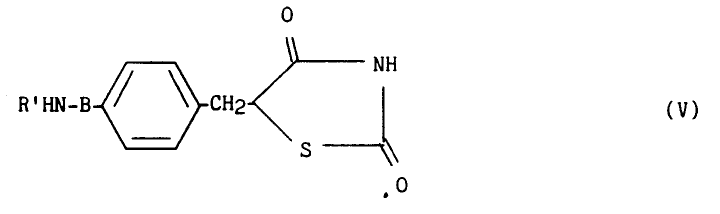 dans laquelle :
      - B a la signification précédemment définie et
      - R' représente un atome d'hydrogène ou un radical alkyle de 1 à 5 atomes de carbone en chaine droite ou ramifiée.

La méthode A, décrite par T. Sohda et coll, Chem. Pharm. Bull., 30 (10) 3580-3600 (1982), a été mise en oeuvre de façon particulièrement adéquate pour préparer les composés I en opérant dans un solvant approprié tel que, par exemple, le méthanol, l'éthanol, le sulfolane®, le diméthylformamide et en chauffant à 100-120 °C pendant 6 à 10 heures le mélange de composé II et de thiourée, puis en hydrolysant le composé III au moyen d'un acide fort, HCl ou H₂SO₄ par exemple.

Les matières premières de formule II ont été préparées à partir des amines de formule : 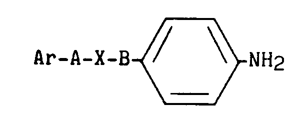 elles-mêmes obtenues par hydrogénation sous une pression de 3.10⁵ Pa en présence de Nickel de Raney des composés nitrés correspondants.

La méthode B est avantageusement réalisée en effectuant la réaction des composés IV et V à une température de 20-25 °C pendant 8 à 15 heures dans un solvant adéquat tel que par exemple le tétrahydrofurane, le diméthylformamide ou l'acétonitrile, seuls ou en mélange, en présence d'un accepteur de l'acide formé au cours de la réaction.

Cet accepteur peut-être par exemple la triéthylamine ou un excès du composé V utilisé pour la synthèse.

Les produits I, ainsi préparés selon les méthodes A ou B, peuvent être purifiés par cristallisation au sein des solvants organiques usuels appropriés ou par chromatoflash sur support de silice Amicon (35-70 µ) en éluant avec des solvants appropriés tels que : CH₂Cl₂, CH₂Cl₂- acétone (95-5), toluène-éthanol (95-5), sous une pression de 0,5 à 1 atmosphère d'azote.

Les composés de formule I peuvent être transformés en sels d'addition avec les acides, sels qui font, à ce titre, partie de l'invention. Comme acides utilisables pour la formation de ces sels, on peut citer par exemple, dans la série minérale, les acides chlorhydrique, bromhydrique, nitrique, sulfurique, phosphorique et, dans la série organique, les acides acétique, propionique, maléique, fumarique, tartrique, oxalique, benzoîque, méthanesulfonique et iséthionique.

Les exemples suivants illustrent la présente invention. Les points de fusion sont déterminés à la platine chauffante de Kofler, sauf mention contraire.

### Exemple de préparation 1

5-{4-[2-(2-méthoxy 5-chloro benzamido) éthyl] benzyl} thiazolidine 2,4-dione.

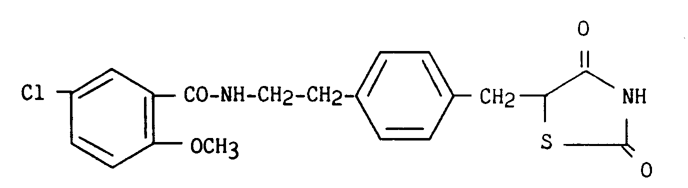

On chauffe pendant 10 heures à 120 °C un mélange de 4,1 g de 3-{4-[2-(2-méthoxy-5-chloro benzamido) éthyl] phényl}-2-chloro propionate de méthyle (huile) et 1,52 g de thiourée dans 40 ml de sulfolane®. L'imine formée est hydrolysée en ajoutant au mélange 14 ml d'HCl, 2N et en chauffant l'ensemble à 100 °C pendant 8 heures. Lorsque la réaction est terminée, on dilue avec 600 ml d'eau et décante la partie aqueuse. On extrait le produit gommeux avec CH₂Cl₂ et lave à l'eau la solution organique. Après évaporation du solvant, le résidu est purifié sur 90 g de silice par chromatoflash en éluant avec le mélange CH₂Cl₂-méthanol (95-5). Les éluats évaporés donnent 2,6 g du produit attendu, sous forme cristallisée, PF : 182 °C.

### Exemple de préparation 2

(R,S) 5-{4-[2-(4-hydroxy-3,5-ditert-butyl benzamido) éthyl] benzyl} thiazolidine-2,4-dione.

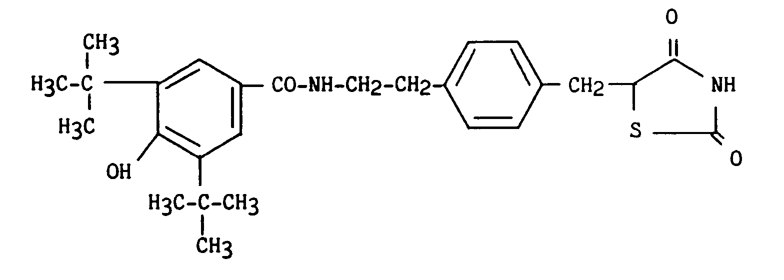

A une solution de 3 g d'acide 4-hydroxy-3,5-ditert-butyl benzoïque dans 150 ml de tétrahydrofurane anhydre, on ajoute 1,9 g de 1,1-carbonyl diimidazole et on agite la solution pendant 8 heures à température ambiante. Ensuite, on ajoute 3 g de 5-[4-(2-amino éthyl) benzyl] thiazolidine-2,4-dione (fondant à 200 °C) et laisse le mélange sous agitation à température ambiante pendant 15 heures. Le solvant est ensuite évaporé et le résidu est repris avec un mélange à 10 % de Na₂CO₃ dans CH₂Cl₂. Le précipité est filtré puis purifié par chromatoflash sur 180 g de silice, en éluant avec le mélange CH₂Cl₂-méthanol (97-3).

Après évaporation des éluats, on recueille 3 g de produit attendu sous forme amorphe. La 5-[4-(2-aminoéthyl) benzyl] thiazolidine-2,4-dione de départ a été préparée selon la méthode de SOHDA et Coll. à partir du 3-[4-(2-acétamido éthyl) phényl]-2-chloro propionate de méthyle (huile).

### Exemple de préparation 3

(R,S) 5-{4-[2-(salicyloylamino) éthyl] benzyl} thiazolidine-2,4-dione.

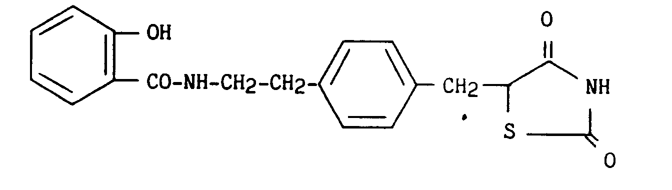

A une solution de 3 g de 5-[4-(2-aminoéthyl) benzyl] thiazolidine-2,4-dione et 1,16 g de triéthylamine dans 50 ml de diméthylformamide anhydre, on ajoute 2,3 g de chlorure d'acétyl salicyloyle et laisse agiter le mélange à température ordinaire pendant 15 heures.
Le diméthylformamide est ensuite évaporé et le résidu repris avec CH₂Cl₂ et eau.
La phase organique est décantée puis évaporée sous pression réduite. On obtient 5,3 g d'un résidu huileux que l'on chromatographie sur 170 g de silice en éluant avec le mélange CH₂Cl₂-acétone (90-10). L'évaporation des éluats laisse 3 g de produit amorphe que l'on dissout dans 50 ml d'éthanol puis saponifie en présence de 8,5 ml de NaOH, N, à température ordinaire pendant 5 heures. On évapore ensuite le solvant, reprend le produit avec HCl, N et essore les cristaux blancs formés.
On obtient 1,9 g du produit attendu. PF : 184 °C.

### Exemples 1 et 2 :

En opérant par analogie avec les méthodes ci-dessus exemplifiées, ont été préparés les composés suivants :
1) (R,S) 5-{4-[2-(indol-2-yl carbonylamino)éthyl]benzyl}thiazolidine-2,4-dione, PF : 256 °C.
2) (R,S) 5-{4-[2-(3-(3,4-méthylènedioxyphényl)prop-2-énoylamino)éthyl] benzyl} thiazolidine-2,4-dione, PF (cap) : 155-157 °C.

### ETUDE PHARMACOLOGIQUE

### A/ Etude de l'activité des composés de l'invention sur un modèle de diabète non insulino-dépendant (NIDDM).

Les animaux (souris ob/ob) sont traités chaque jour, pendant 4 jours, en administrant par voie orale, les composés de l'invention en suspension dans une solution à 20 % de gomme du Sénégal.
Avant et après traitement, soit à J0 et à J5, le sang est prélevé par ponction du sinus orbital et la glycémie est déterminée.
Le tableau 1 présente les doses des différents produits à administrer pour obtenir le même effet hypoglycémiant (base 100).

**Tableau 1**

| **doses provoquant le même effet hypoglycémiant chez la souris ob/ob** | | |
|---|---|---|
| **Produits** | **Dose mg/kg/jour pendant 4 jours** | **Pouvoir hypoglycémiant** |
| Ciglitazone | 50-100 | 100 |
| Exemple de préparation 1 | ≦ 10 | 100 |
| Exemple 2 | 10 | 100 |

### B/ Etude de l'activité des composés de l'invention sur un modèle de tolérance au glucose diminuée associée à une hyperinsulinémie et une hyperlipémie .

Les animaux (rats Zucker Fa/Fa mâles) sont traités, chaque jour, pendant 10 jours, en administrant, par voie orale, les composés de l'invention à la dose de 5 mg/kg/jour en suspension dans une solution à 20 % de gomme du Sénégal. Le 11ème jour, les animaux sont sacrifiés et le sang recueilli afin de déterminer la glycémie, les triglycérides plasmatiques et l'insuline immuno-réactive. D'autre part, les animaux sont pesés avant et après traitement.

Dans ces conditions, les composés de l'invention n'ont pas d'influence sur le taux de glucose circulant mais diminuent le taux de triglycérides (TG) et d'acides gras libres plasmatiques (AGL) ainsi que celui de l'insuline immuno-réactive. Cette activité est égale ou supérieure à celle d'autres dérivés de thiazolidinedione de référence.

**Tableau 2**

| **Etude pharmacologique sur rats mâles Zucker Fa/Fa** | | | | | | |
|---|---|---|---|---|---|---|
| | **Dose (mg/kg/jour) pendant 10 jours** | **Poids ΔP% J**_{**11**}**-J**_{**1**} | **Glycémie (%)** | **Plasma insuline (%)** | **TG (%)** | **AGL (%)** |
| **Contrôle** | 0 | +100 | 100 | 100 | 100 | 100 |
| **Pioglitazone** | 5 | +320 | 115 | 49 | 35 | 57 |
| **Exemple de préparation 1** | 5 | +112 | 113 | 71 | 94 | 83 |

Administrés per os à des rats SDCD mâles pesant 175 g les produits sont sans effet sur les éléments figurés du sang et le volume plasmatique, à la différence de la pioglitazone :

**Tableau 3**

| **(valeurs contrôle = 100)** | | | | |
|---|---|---|---|---|
| **Durée de traitement : 8 jours** | **Globules blancs** | **Globules rouges** | **Hématocrite** | **Hémoglobine** |
| Pioglitazone : 100 mg/kg/j | - 46 % | - 37 % | - 26 % | - 29 % |

| Exemple de préparation 1 | | | | |
|---|---|---|---|---|
| 100mg/kg/j | - 6 % | - 2 % | + 11 % | + 11 % |
| 250 mg/kg/j | - 4 % | - 11 % | + 4 % | + 4 % |

## Revendications

1. Les composés de thiazolidine dione de formule I : 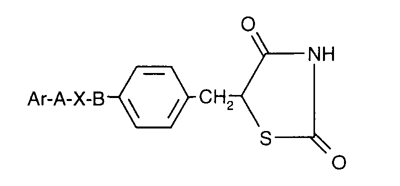 dans laquelle :
**Ar** représente un radical hétérobicyclique, renfermant 1 ou 2 héteroatomes choisis parmi les atomes d'azote, d'oxygène ou de soufre, éventuellement substitué par un radical oxo et/ou par un ou plusieurs atomes d'halogène, radicaux alkyle (ayant de 1 à 5 atomes de carbone) ou phényle lui-même éventuellement substitué par un ou plusieurs atomes d'halogène, radicaux trifluorométhyle, alkyle ou alkoxy ayant chacun de 1 à 5 atomes de carbone;
**A** représente une liaison simple, une chaine hydrocarbonée de 2 à 3 atomes de carbone renfermant une double liaison, ou une chaine de formule :
―(CH₂)ₘ― , 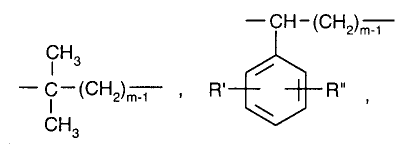 ―O―(CH₂)ₘ― , ―S―(CH₂)ₘ―
dans lequelles m est un nombre entier de 1 à 3 et R' et R'', identiques ou différents, représentent chacun un atome d'hydrogène ou un radical alkyle ayant de 1 à 5 atomes de carbone en chaine droite ou ramifiée;
**X** représente -CONR- ou -SO₂NR- dans lesquelles R représente un atome d'hydrogène ou un radical alkyle de 1 à 5 atomes de carbone en chaine droite ou ramifiée renfermant éventuellement une double liaison ;
**B** représente une chaine hydrocarbonée saturée de 1 à 6 atomes de carbone éventuellement ramifiée et/ou substituée par un radical hydroxy ou un radical oxo;
et leurs énantiomères et diastéréoisomères, ainsi que leurs sels d'addition à un acide pharmaceutiquement acceptable.

2. Le procédé de préparation des composés de la revendication 1, caractérisé en ce que :
- soit,
A) l'on fait réagir un composé de formule II : 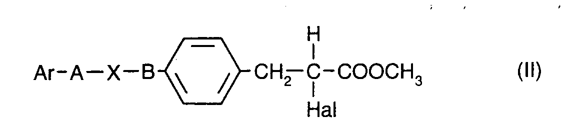 dans laquelle :
**Ar**, **A**, **X** et **B** ont les significations définies dans la revendication 1 et
**Hal** représente un atome de chlore ou de brome
avec la thiourée 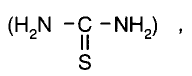 et l'on hydrolyse l'imine ainsi obtenue de formule III : 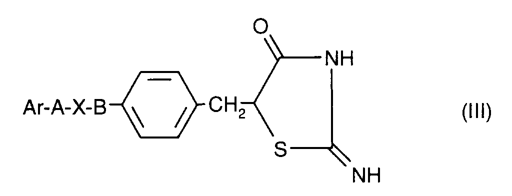 dans laquelle **Ar**, **A**, **X** et **B** ont les significations précédémment définies;
- soit
B) l'on fait réagir un composé de formule IV :
Ar-A-X' (IV)
dans laquelle :
**Ar** et **A** ont les significations définies dans la revendication 1, et
**X'** représente COCl, SO₂Cl, un ester activé ou CO-O-CO-A-Ar,
avec un composé de formule V : 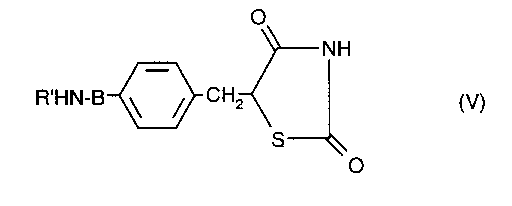 dans laquelle :
**B** a la signification définie dans la revendication 1 et
**R'** représente un atome d'hydrogène ou un radical alkyle de 1 à 5 atomes de carbone en chaine droite ou ramifiée ;
et, si on le désire, on sépare les isomères des composés ainsi obtenus au moyen des méthodes classiques de séparation et/ou on transforme les dits composés en leurs sels d'addition à un acide pharmaceutiquement acceptable.

3. Les compositions pharmaceutiques contenant comme principe actif au moins un composé selon la revendication 1, seul ou en combinaison avec un ou plusieurs excipients pharmaceutiques appropriés.

4. Les compositions pharmaceutiques selon la revendication 3 utiles dans le traitement des états d'insulino-résistance et/ou des diabètes non insulinopéniques associés ou non à une hypertension.

## Claims

1. Thiazolidinedione compounds of formula I : 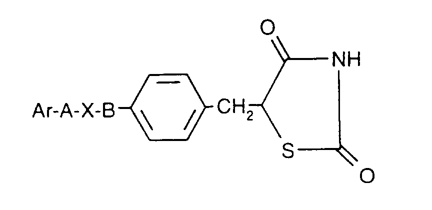 wherein :
**Ar** represents a heterobicyclic radical containing 1 or 2 hetero atoms selected from nitrogen, oxygen and sulphur atoms, which is optionally substituted by an oxo radical and/or by one or more halogen atoms, alkyl (having from 1 to 5 carbon atoms) or phenyl radicals, phenyl itself optionally being substituted by one or more halogen atoms, trifluoromethyl radicals, alkyl or alkoxy radicals each having from 1 to 5 carbon atoms ;
**A** represents a single bond, a hydrocarbon chain having 2 or 3 carbon atoms and including a double bond, or a chain of the formula
-(CH₂)ₘ-, 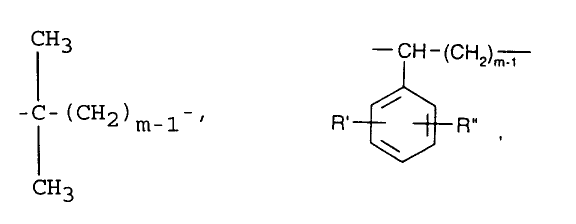 -O-(CH₂)ₘ- or -S-(CH₂)ₘ- wherein m is an integer from 1 to 3 and R' and R'', which are the same or different, each represents a hydrogen atom or a straight-chain or branched alkyl radical having from 1 to 5 carbon atoms;
**X** represents -CONR- or -SO₂NR- wherein R represents a hydrogen atom or a straight-chain or branched alkyl radical having from 1 to 5 carbon atoms and optionally including a double bond;
**B** represents a saturated hydrocarbon chain having from 1 to 6 carbon atoms which is optionally branched and/or substituted by a hydroxy radical or an oxo radical;
and their enantiomers and diastereoisomers, as well as their addition salts with a pharmaceutically acceptable acid.

2. A process for the preparation of the compounds of claim 1, characterised in that :
- either,
A) a compound of formula II : 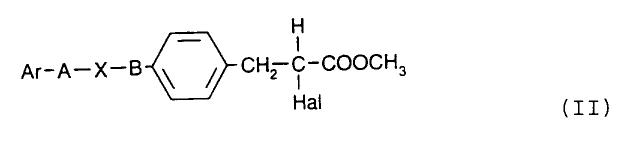 wherein :
**Ar**, **A**, **X** and **B** are as defined in claim 1 and
**Hal** represents a chlorine or bromine atom,
is reacted with thiourea 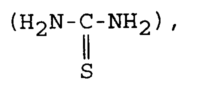 and the imine so obtained of formula III : 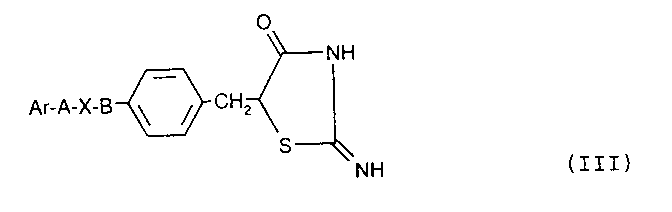 wherein **Ar**, **A**, **X** and **B** are as defined hereinbefore, is hydrolysed;
- or,
B) a compound of formula IV :
Ar-A-X' (IV)
wherein :
**Ar** and **A** are as defined in claim 1 and
**X'** represents COCl, SO₂Cl, an activated ester or CO-O-CO-A-Ar,
is reacted with a compound of formula V : 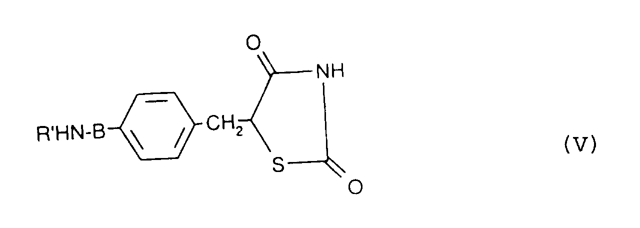 wherein :
**B** is as defined in claim 1 and
**R'** represents a hydrogen atom or a straight-chain or branched alkyl radical having from 1 to 5 carbon atoms;
and, if desired, the isomers of the compounds so obtained are separated by conventional methods of separation and/or the said compounds are converted into their addition salts with a pharmaceutically acceptable acid.

3. Pharmaceutical compositions containing as active ingredient at least one compound according to claim 1, alone or in combination with one or more appropriate pharmaceutical excipients.

4. Pharmaceutical compositions according to claim 3 for use in the treatment of conditions of insulin resistance and/or non-insulinopenic diabetes which may or may not be combined with hypertension.

## Patentansprüche

1. Thiazolidindion-Verbindungen der Formel I: 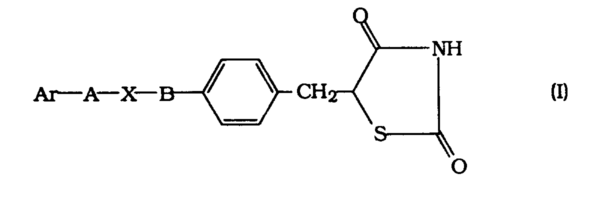 in der
**Ar** eine heterobicyclische Gruppe darstellt, die 1 oder 2 Heteroatome ausgewählt aus Stickstoff-, Sauerstoff- oder Schwefelatomen umfaßt und gegebenenfalls durch eine Oxo- und/oder eines oder mehrere Halogenatome, Alkylgruppen (mit 1 bis 5 Kohlenstoffatomen) oder Phenylgruppen substituiert ist, die ihrerseits gegebenenfalls durch eines oder mehrere Halogenatome, Trifluormethylgruppen, Alkylgruppen oder Alkoxygruppen mit jeweils 1 bis 5 Kohlenstoffatomen substituiert ist;
**A** eine Einfachbindung, eine Kohlenwasserstoffkette mit 2 bis 3 Kohlenstoffatomen, die eine Doppelbindung umfaßt, oder eine Kette der Formel darstellt:
―(CH₂)ₘ― , 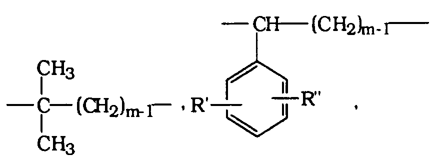 ―O―(CH₂)ₘ―, ―S―(CH₂)ₘ―
in denen m eine ganze Zahl mit einem Wert von 1 bis 3 und R' und R'', die gleichartig oder verschieden sind, jeweils ein Wasserstoffatom oder eine Alkylgruppe mit 1 bis 5 Kohlenstoffatomen in gerader oder verzweigter Kette bedeuten;
**X** -CONR- oder-SO₂NR- darstellt, worin Rein Wasserstoffatom oder eine Alkylgruppe mit 1 bis 5 Kohlenstoffatomen in gerader oder verzweigter Kette, die gegebenenfalls eine Doppelbindung aufweist, bedeutet;
**B** eine gesättigte Kohlenwasserstoffkette mit 1 bis 6 Kohlenstoffatomen, die gegebenenfalls verzweigt und/oder durch eine Hydroxygruppe oder eine Oxogruppe substituiert ist, darstellt;
und deren Enantiomere und Diastereoisomere sowie deren Additionssalze mit einer pharmazeutisch annehmbaren Säure.

2. Verfahren zur Herstellung der Verbindungen nach Anspruch 1, dadurch gekennzeichnet, daß man:
- entweder
A) eine Verbindung der Formel II: 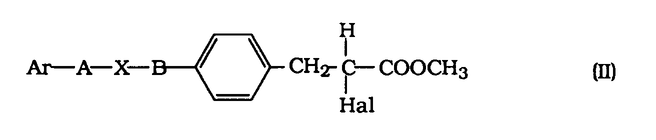 in der:
**Ar**, **A**, **X** und **B** die in Anspruch 1 angegebenen Bedeutungen besitzen und
**Hal** ein Chloratom oder ein Bromatom darstellt,
mit Thioharnstoff 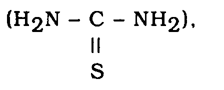 umsetzt und das in dieser Weise erhaltene Imin der Formel III: 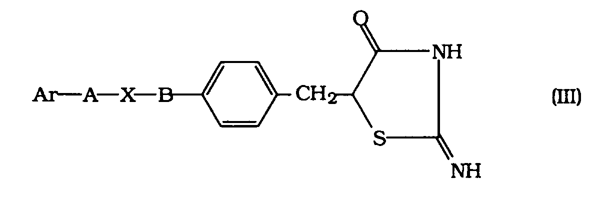 in der **Ar**, **A**, **X** und **B** die oben angegebenen Bedeutungen besitzen, hydrolysiert;
- oder
B) eine Verbindung der Formel IV:
Ar - A - X' (IV)
in der:
**Ar** und **A** die in Anspruch 1 angegebenen Bedeutungen besitzen und
**X'** COCl, SO₂Cl, einen aktivierten Ester oder CO-O-CO-A-Ar darstellt,
mit einer Verbindung der Formel V: 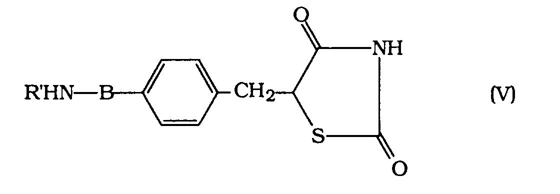 in der:
**B** die in Anspruch 1 angegebenen Bedeutungen besitzt und
**R'** ein Wasserstoffatom oder eine Alkylgruppe mit 1 bis 5 Kohlenstoffatomen in gerader oder verzweigter Kette bedeutet, umsetzt;
und gewünschtenfalls die Isomeren der in dieser Weise erhaltenen Verbindungen mit Hilfe klassischer Trennverfahren auftrennt und/oder die genannten Verbindungen in ihre Additionssalze mit einer pharmazeutisch annehmbaren Säure überführt.

3. Pharmazeutische Zubereitungen enthaltend als Wirkstoff mindestens eine Verbindung nach Anspruch 1 allein oder in Kombination mit einem oder mehreren pharmazeutisch geeigneten Trägermaterialien.

4. Pharmazeutische Zubereitungen nach Anspruch 3 zur Behandlung von Insulino-Resistenzzuständen und/oder nicht-insolinopenen Diabeteszuständen, welche gegebenenfalls mit einer Hypertension verbunden sind.
